# EUROPEAN PATENT APPLICATION

(11) **EP 3 231 418 A1**
(43) Date of publication of application: **18.10.2017**
(21) Application number: 16165247.4
(22) Date of filing: 14.04.2016
(51) Int. Cl.: A61K 9/16, A61K 31/495

(54) **GRANULE FORMULATION FOR ORAL ADMINISTRATION**

(71) Applicant: Richter Gedeon Nyrt., 1103 Budapest (HU)
(72) Inventor: Yasuhi, Imada, Osaka-city, 541-8505 (JP); Fuminori, Ozaki, Osaka-city, 541-8505 (JP); Akihiro, Suzuki, Osaka-shi Osaka, 541-8505 (JP); Keiji, Mitsui, Osaka-city, 541-8505 (JP)
(74) Representative: Gullerné Lados, Zsuzsanna

(57) **Abstract**

The invention relates to a granule formulation that enables cariprazine hydrochloride to be stably stored. The solution provides a granule formulation containing cariprazine hydrochloride, wherein said granule formulation contains lactose as a primary diluent.

## Description

### Field of the Invention

The present invention relates to a granule formulation of cariprazine hydrochloride for oral administration that is effective as a therapeutic drug for schizophrenia, etc.

### Background of the Invention

In patent document WO2005012266, it is stated that cariprazine hydrochloride is effective as a therapeutic drug for schizophrenia, etc.

Furthermore, in patent document WO2009104739 a solid formulation for oral administration that contains cariprazine hydrochloride is disclosed. Although solid formulations for oral administration that contain cariprazine hydrochloride have become known primarily in the form of tablets in the prior art, not enough knowledge has been obtained of other dosage forms, in particular granules, fine granules or powders, that have superior properties in formulations.

The problem of the present invention is to provide a granule formulation that contains cariprazine hydrochloride.

The present inventors diligently investigated granule formulations containing cariprazine hydrochloride, in particular granules, fine granules or powders. They found that, when a large amount of crystalline cellulose was used as a diluent, large quantities of impurities were produced, especially under high temperatures, but when lactose was used as the primary diluent, cariprazine hydrochloride could be stored stably. In addition, granules, fine granules, or powders that contained cariprazine hydrochloride tended to easily aggregate with increasing humidity, but it was found that aggregation could be inhibited by adding a fluidizer. This finding completed the present invention.

### Brief description of the invention

In other words, the present invention is as follows.
1) A granule formulation containing cariprazine hydrochloride for oral administration, wherein said granule formulation uses a diluent that contains lactose, crystalline cellulose or starch, with the percentage content of the total quantity of diluent being 70 to 100 wt% of lactose, 0 to 25 wt% of crystalline cellulose, and 0 to 5 wt% of starch.
2) A granule formulation for oral administration, wherein said granule formulation consists of granules, fine granules or powders described in 1).
3) A granule formulation for oral administration described in 1) or 2), wherein said granule formulation contains one or more types of binder selected from hydroxypropyl cellulose, hypromellose, ethyl cellulose, methyl cellulose, povidone and polyvinyl alcohol.
4) A granule formulation for oral administration described in any one of 1) to 3), wherein said granule formulation contains one or more types of fluidizer at a percentage content of 0 to 5 wt%, selected from light anhydrous silicic acid, hydrous silicon dioxide, stearic acid, calcium stearate, magnesium stearate, sodium stearyl fumarate, sugar fatty acid ester, titanium oxide, talc, dibasic calcium phosphate or its hydrate, corn starch, magnesium aluminometasilicate and sodium lauryl sulfate.
5) A granule formulation for oral administration described in 4), wherein said granule formulation is obtained by mixing together cariprazine hydrochloride, a diluent and a binder, granulating the mixture, then mixing the granulated product in a fluidizer.

### Detailed description of the Invention

In the present invention, the blending quantity of cariprazine hydrochloride is usually 0.01 to 70 wt%, preferably 0.1 to 50 wt% and more preferably 0.4 to 10 wt%, of the total formulation.

The "primary diluent" refers to a diluent that makes up 50 wt% or more of the total amount of diluent. In the granule formulation of the present invention, lactose is used as the primary diluent. Lactose may also be used in its hydrate form. Other diluents that can be used are crystalline cellulose and starch (corn starch, potato starch, wheat starch, rice starch, partially pregelatinized starch, perforated starch, etc.), etc. Preferable diluents are: (a) Regarding the percentage content of the total amount of diluent, a diluent consisting 50 to 100 wt% of lactose, 0 to 50 wt% of methyl cellulose, and 0 to 35 wt% of starch; (b) Regarding the percentage content of the total amount of diluent, a diluent consisting 60 to 100 wt% of lactose, 0 to 29 wt% of crystalline cellulose, and 0 to 11 wt% of starch; (c) Regarding the percentage content of the total amount of diluent, a diluent consisting 70 to 100 wt% of lactose, 0 to 25 wt% of crystalline cellulose, and 0 to 5 wt% of starch; (d) Regarding the percentage content of the total amount of diluent, a diluent consisting 80 to 95 wt% of lactose, 5 to 20 wt% of methyl cellulose, and 0% of starch; (e) Regarding the percentage content of the total amount of diluent, a diluent consisting 95 to 99 wt% of lactose, 1 to 5 wt% of starch, and 0% of methyl cellulose; and (f) a diluent that contains only lactose (regarding the percentage content of the total amount of diluent, a diluent consisting 100% of lactose). The diluent used can be 5 to 99.9 wt%, preferably 80 to 99 wt%, of the total formulation.

Binders that have been suggested include hydroxypropyl cellulose, hypromellose (hydroxypropyl methylcellulose), ethyl cellulose, methyl cellulose, povidone (polyvinylpyrrolidone), polyvinyl alcohol, powdered acacia, gelatin, and pullulan, preferably hydroxypropyl cellulose, etc. These binders can account for 0.5 to 10 wt%, preferably 1 to 5 wt%, of the total formulation.

Fluidizers that have been suggested include light anhydrous silicic acid, hydrous silicic acid, stearic acid, calcium stearate, magnesium stearate, sodium stearyl fumarate, sugar fatty acid ester, titanium oxide, talc, dibasic calcium phosphate or its hydrate, corn starch, magnesium aluminometasilicate, sodium lauryl sulfate, crystalline cellulose, hydrogenated oil, etc., preferably light anhydrous silicic acid, hydrous silicic acid, calcium stearate, magnesium stearate, titanium oxide, etc., and more preferably calcium stearate, magnesium stearate, etc. These fluidizers can account for 0 to 5 wt%, preferably 1 to 3 wt%, of the total formulation.

Judging the formulation of the present invention from the standpoint of the patient's feeling when taking it, it is desirable that 90% or more of the formulation consists of particles with a diameter of 500 µm or less. From the standpoint of the patient's feeling when taking the formulation and its ease of handling, it is desirable that at least 85% of the formulation consists of particles with a diameter of 75 to 500 µm.

The formulation of the present invention can be produced by mixing together and granulating, for example, cariprazine hydrochloride and a diluent, and a binder, too, depending on the situation, using instruments such as a high-speed agitation granulator, a fluid bed dryer, a centrifugal tumbling fluidized bed granulating coating machine or kneading machine, together with water or a binder solution (dissolving the above binders in a solvent such as water or ethanol or dispersing them), then mixing a fluidizer into the resulting product.

The present invention will now be further described in detail using the examples below. However, the invention shall not be construed as limited to the particular forms disclosed in these examples.

The high-speed agitation granulator used was a LFS-GS-2J model manufactured by Fukae Powtec Corporation.

The fluid bed dryer was a MP-01/03 model manufactured by Powrex Corporation or a FLO-5 or FLO-15 model manufactured by Freund Corporation. The sizing machine was a P-02S model manufactured by Dulton Co., Ltd. or a QC-197S model manufactured by Powrex Corporation. The mixer was a TCW-30 model manufactured by Tokuju Corporation.

### Example 1

Cariprazine hydrochloride 4.36 g, lactose hydrate 383.64 g and hydroxypropyl cellulose 12 g were placed in a high-speed agitation granulator, water was added, and the mixture was granulated. The granulated product was dried in a fluid bed dryer, yielding a dried powder containing cariprazine 1 wt%. The obtained dried powder was sized in a sizing machine using a 0.5 mm mesh screen to obtain a sized powder.

### Example 2

Cariprazine hydrochloride 4.36 g, lactose hydrate 383.64 g and hypromellose 12 g were placed in a high-speed agitation granulator, water was added, and the mixture was granulated. The granulated product was dried in a fluid bed dryer, yielding a dried powder containing cariprazine 1 wt%. The obtained dried powder was sized in a sizing machine using a 0.5 mm mesh screen to obtain a sized powder.

### Example 3

Magnesium stearate 1 g was added to 50 g of the sized powder obtained in Example 1, and the mixture was mixed 100 times in a plastic bag to obtain a mixed powder.

### Example 4

Cariprazine hydrochloride 163.5 g and lactose hydrate 14,086.5 g were placed in a fluid bed dryer, a 6% aqueous solution of hydroxypropyl cellulose was sprayed onto it and the mixture was granulated. The granulated product was sized in a sizing machine using a 0.6 mm mesh screen to obtain a sized powder.

### Example 5

Magnesium stearate 0.35 g was added to 68.6 g of the sized powder obtained in Example 4, and the mixture was shaken 200 times in a glass bottle to obtain a mixed powder.

### Example 6

Magnesium stearate 0.70 g was added to 68.6 g of the sized powder obtained in Example 4, and the mixture was shaken 200 times in a glass bottle to obtain a mixed powder.

### Example 7

Magnesium stearate 1.40 g was added to 68.6 g of the sized powder obtained in Example 4, and the mixture was shaken 200 times in a glass bottle to obtain a mixed powder.

### Example 8

Magnesium stearate 2.10 g was added to 68.6 g of the sized powder obtained in Example 4, and the mixture was shaken 200 times in a glass bottle to obtain a mixed powder.

### Example 9

Cariprazine hydrochloride 163.5 g and lactose hydrate 14,086.5 g were placed in a fluid bed dryer, a 6% aqueous solution of hydroxypropyl cellulose was sprayed onto it and the mixture was granulated. The granulated product was sized in a sizing machine using a 0.6 mm mesh screen to obtain a sized powder. Magnesium stearate 254.3 g was added to 12,460 g of the obtained sized powder, and the mixture was mixed for 10 minutes in a blender to obtain a mixed powder containing cariprazine 1 wt%.

### Comparative Example 1

Cariprazine hydrochloride 4.36 g, lactose hydrate 343.64 g, crystalline cellulose 40 g and hydroxypropyl cellulose 12 g were placed in a high-speed agitation granulator, water was added, and the mixture was granulated. The granulated product was dried in a fluid bed dryer, yielding a dried powder containing cariprazine 1 wt%. The obtained dried powder was sized in a sizing machine using a 0.5 mm mesh screen to obtain a sized powder.

### Comparative Example 2

Light anhydrous silicic acid 1 g was added to 100 g of the sized powder obtained in Comparative Example 1, and the mixture was mixed 100 times in a plastic bag to obtain a mixed powder.

### Comparative Example 3

Magnesium stearate 2 g was added to 100 g of the sized powder obtained in Comparative Example 1, and the mixture was mixed 100 times in a plastic bag to obtain a mixed powder.

### Comparative Example 4

Cariprazine hydrochloride 4.36 g, lactose hydrate 343.64 g, crystalline cellulose 40 g and hypromellose 12 g were placed in a high-speed agitation granulator, water was added, and the mixture was granulated. The granulated product was dried in a fluid bed dryer, yielding a dried powder containing cariprazine 1 wt%. The obtained dried powder was sized in a sizing machine using a 0.5 mm mesh screen to obtain a sized powder.

The various stabilities of the fine granules obtained in the above Examples and Comparative Examples were evaluated.

Furthermore, in the above Examples and Comparative Examples, the granules, fine granules or powders were appropriately manufactured using mesh screens whose size had been appropriately adjusted when a dried powder was sized in a sizing machine.

### Stability Test Example 1

The fine granules obtained in Example 1, Example 2, Example 3, Comparative Example 1, Comparative Example 2, Comparative Example 3 and Comparative Example 4 were subdivided into vials, stored in a hermetically stopped state at a temperature of 60°C in a thermostatic oven for 2 weeks and 4 weeks, then subjected to stability testing. The purity of each of these samples was confirmed in high-performance liquid chromatography, and the impurities at each time point were observed (Table 1). No obvious increase in impurities as a result of storage was observed in Example 1, Example 2 and Example 3, but marked increases in the maximum quantity of individual impurities and the total quantity of impurities were observed in Comparative Example 1, Comparative Example 2, Comparative Example 3 and Comparative Example 4. Furthermore, the term "maximum quantity of individual impurities" in the present Specification means the maximum number of impurities produced among each of the impurities produced at each time point.

**Table 1**

| | Length of storage | Maximum individual impurities (%) | Total quantity of impurities (%) |
|---|---|---|---|
| Example 1 | At start of test | 0.02 | 0.02 |
| | 2 weeks | 0.03 | 0.05 |
| | 4 weeks | 0.03 | 0.05 |
| Example 2 | At start of test | 0.02 | 0.04 |
| | 2 weeks | 0.04 | 0.14 |
| | 4 weeks | 0.04 | 0.12 |
| Example 3 | At start of test | 0.02 | 0.02 |
| | 2 weeks | | |
| | 4 weeks | 0.04 | 0.10 |
| Comparative Example 1 | At start of test | 0.02 | 0.02 |
| | 2 weeks | 0.12 | 0.35 |
| | 4 weeks | 0.94 | 1.69 |
| Comparative Example 2 | At start of test | 0.04 | 0.09 |
| | 2 weeks | 0.18 | 0.50 |
| | 4 weeks | 1.09 | 2.26 |
| Comparative Example 3 | At start of test | 0.02 | 0.02 |
| | 2 weeks | 0.17 | 0.56 |
| | 4 weeks | 0.76 | 1.64 |
| Comparative Example 4 | At start of test | 0.02 | 0.04 |
| | 2 weeks | 1.03 | 1.83 |
| | 4 weeks | 1.08 | 2.41 |

### Stability Test Example 2

The fine granules obtained in Example 1, Example 2, Comparative Example 1 and Comparative Example 3 were subdivided into vials, stored in an open state in a thermo hygrostat chamber at a temperature of 40°C and a relative humidity of 75% for 4 weeks, 3 months and 6 months, then subjected to stability testing. The purity of each of these samples was confirmed in high-performance liquid chromatography, and the impurities at each time point were observed (Table 2). No obvious increase in impurities with the passage of time was observed in Example 1, Example 2 Comparative Example 1 and Comparative Example 3.

**Table 2**

| | Length of storage | Maximum individual impurities (%) | Total quantity of impurities (%) |
|---|---|---|---|
| Example 1 | At start of test | 0.02 | 0.02 |
| | 4 weeks | 0.02 | 0.04 |
| | 3 months | 0.02 | 0.06 |
| | 6 months | 0.03 | 0.07 |
| Example 2 | At start of test | 0.02 | 0.04 |
| | 4 weeks | 0.02 | 0.02 |
| | 3 months | 0.02 | 0.06 |
| | 6 months | | |
| Comparative Example 1 | At start of test | 0.02 | 0.02 |
| | 4 weeks | 0.03 | 0.07 |
| | 3 months | 0.05 | 0.11 |
| | 6 months | 0.06 | 0.17 |
| Comparative Example 3 | At start of test | 0.02 | 0.02 |
| | 4 weeks | 0.02 | 0.04 |
| | 3 months | 0.02 | 0.06 |
| | 6 months | 0.03 | 0.14 |

### Stability Test Example 3

The fine granules obtained in Example 1, Example 2, Example 3, Comparative Example 1, Comparative Example 2, Comparative Example 3 and Comparative Example 4 were subdivided into vials, stored in an open state in a thermo hygrostat chamber at a temperature of 40°C and a relative humidity of 75% for 4 weeks, then subjected to stability testing. The purity of each of these samples was confirmed in high-performance liquid chromatography, and the impurities at each time point were observed (Table 3).

In Example 1, Example 2, Example 3, Comparative Example 1, Comparative Example 2, Comparative Example 3 and Comparative Example 4, no significant differences were observed in the quantity of impurities at the start of testing and after 4 weeks of storage.

**Table 3**

| | Length of storage | Maximum individual impurities (%) | Total quantity of impurities (%) |
|---|---|---|---|
| Example 1 | At start of test | 0.02 | 0.02 |
| | 4 weeks | 0.02 | 0.04 |
| Example 2 | At start of test | 0.02 | 0.04 |
| | 4 weeks | 0.02 | 0.02 |
| Example 3 | At start of test | 0.02 | 0.02 |
| | 4 weeks | 0.02 | 0.06 |
| Comparative Example 1 | At start of test | 0.02 | 0.02 |
| | 4 weeks | 0.03 | 0.07 |
| Comparative Example 2 | At start of test | 0.02 | 0.02 |
| | 4 weeks | 0.03 | 0.07 |
| Comparative Example 3 | At start of test | 0.02 | 0.02 |
| | 4 weeks | 0.02 | 0.04 |
| Comparative Example 4 | At start of test | 0.02 | 0.04 |
| | 4 weeks | 0.03 | 0.07 |

As can be seen from the results shown in Table 1, Table 2 and Table 3 that were obtained in Examples 1, 2 and 3, virtually no increase, or a mild increase, was observed in the increase in impurities over time with storage either at a temperature of 40°C and a relative humidity of 75% or at a temperature of 60°C. In contrast, in Comparative Example 1, Comparative Example 2, Comparative Example 3 and Comparative Example 4, a sharp increase in impurities was observed with storage at a temperature of 60°C, even though virtually no increase or a mild increase in impurities was seen with storage at a temperature of 40°C and a relative humidity of 75%.

Based on the above findings, it was found that, even though crystalline cellulose and light anhydrous silicic acid were not used as additives, stability testing of the fine granules obtained in the invention revealed that the chemical stability of cariprazine hydrochloride could be maintained under various storage conditions.

### Example 4 Stability Test

The fine granules obtained in Example 4, Example 5, Example 6, Example 7 and Example 8 were subdivided into vials, which were stored in an open state at a temperature of 25°C and a relative humidity of 75%, at a temperature of 30°C and a relative humidity of 75%, and at a temperature of 40°C and a relative humidity of 75%, and in a hermetically stopped state at a temperature of 40°C and a relative humidity of 75%, in respective thermo hygrostat chambers for 2 weeks and 4 weeks, then subjected to stability testing. The respective formulations were made to flow out of the vials and were visually inspected. In accordance with the assessment criteria shown in Table 4, the state of adherence or aggregation of each formulation was evaluated with a score of 1, 2, 3, 4 or 5 (Table 5).

Aggregation was found to occur more easily as the temperature increased from 25°C to 30°C to 40°C at a constant relative humidity of 75%. However, the addition of a small amount of magnesium stearate led to superior inhibition of aggregation.

**Table 4**

| Score | State |
|---|---|
| 1 | A state of adherence to the vial |
| 2 | A state of adherence remained, even though the formulation came out of the vial when a strong stimulus (blowing, etc.) was applied. |
| 3 | A state of adherence remained, even though the formulation came out of the vial when a mild stimulus (shaking, etc.) was applied. |
| 4 | A state of adherence remained, even though the formulation easily came out of the vial. |
| 5 | A state of no adherence at all. |

**Table 5**

| | Length of storage | 25°C/75% RH Open | 30°C/75% RH Open | 40°C/75% RH Open | 40°C/75% RH Hermetically stopped |
|---|---|---|---|---|---|
| Example 4 | 2 weeks | 3 | 2 | 2 | 5 |
| | 4 weeks | 3 | 2 | 2 | 5 |
| Example 5 | 2 weeks | 3 | 2 | 2 | 5 |
| | 4 weeks | 3 | 2 | 2 | 5 |
| Example 6 | 2 weeks | 4 | 3 | 3 | 5 |
| | 4 weeks | 4 | 3 | 2 | 5 |
| Example 7 | 2 weeks | 4 | 4 | 4 | 5 |
| | 4 weeks | 5 | 4 | 3 | 5 |
| Example 8 | 2 weeks | 4 | 4 | 5 | 5 |
| | 4 weeks | 5 | 4 | 3 | 5 |

### Stability Test Example 5

The fine granules obtained in Example 9 were placed in a resin bottle, which was then stored in an open state or a hermetically stopped state in a thermo hygrostat chamber at a temperature of 40°C and a relative humidity of 75% for 4 weeks, 3 months and 6 months, or was stored in an open state in a thermostatic oven at a temperature of 60°C for 2 weeks and 4 weeks. The fine granules were then subjected to stability testing. The purity of each of these samples was confirmed in high-performance liquid chromatography, and the impurities at each time point were observed (Table 6). In addition, the fine granules were made to flow out of each vial and were visually inspected. In accordance with the assessment criteria shown in Table 4, the state of adherence or aggregation of each formulation was evaluated with a score of 1, 2, 3, 4 or 5 (Table 7).

As can be seen in Table 6, a sharp increase in impurities was not observed under the various storage conditions of Example 9. Superior chemical stability was thus found in this Example.

Moreover, as can be seen in Table 7, no visual change was observed when hermetically stopped storage was used at a temperature of 40°C and a relative humidity of 75%. When storage was open, aggregation was mild. Thus it was confirmed that superior fine granules could be obtained.

**Table 6**

| Example 9 | | | |
|---|---|---|---|
| Storage condition | Length of storage | Maximum individual impurities (%) | Total quantity of impurities (%) |
| - | At start of test | 0.03 | 0.12 |
| 60°C Open | 2 weeks | 0.04 | 0.20 |
| | 4 weeks | 0.05 | 0.20 |
| 40°C/75% RH Open | 4 weeks | 0.03 | 0.10 |
| | 3 months | 0.04 | 0.19 |
| | 6 months | 0.05 | 0.26 |
| 40°C/75% RH Hermetically stopped | 4 weeks | 0.05 | 0.11 |
| | 3 months | 0.05 | 0.21 |
| | 6 months | 0.05 | 0.23 |

**Table 7**

| | Length of storage | 60°C Open | 40°C/75% RH Open | 40°C/75% RH Hermetically stopped |
|---|---|---|---|---|
| Example 9 | At start of test | 5 | | |
| | 2 weeks | | | |
| | 4 weeks | 5 | 4 | 5 |
| | 3 months | | 4 | 5 |
| | 6 months | | 4 | 5 |

The present invention provides a granule formulation, in particular granules, fine granules or powders that enables cariprazine hydrochloride to be stably stored.

## Claims

1. A granule formulation containing cariprazine hydrochloride for oral administration, wherein said granule formulation uses a diluent that contains lactose, crystalline cellulose or starch, with the percentage content of the total quantity of diluent being 70 to 100 wt% of lactose, 0 to 25 wt% of crystalline cellulose, and 0 to 5 wt% of starch.

2. A granule formulation for oral administration according to Claim 1, wherein said granule formulation consists of granules, fine granules or powders.

3. A granule formulation for oral administration according to Claim 1 or Claim 2, wherein said granule formulation contains one or more types of binder selected from hydroxypropyl cellulose, hypromellose, ethyl cellulose, methyl cellulose, povidone and polyvinyl alcohol.

4. A granule formulation for oral administration according to any one of Claims 1 to 3, wherein said granule formulation contains one or more types of fluidizer at a percentage content of 0 to 5 wt%, selected from light anhydrous silicic acid, hydrous silicon dioxide, stearic acid, calcium stearate, magnesium stearate, sodium stearyl fumarate, sugar fatty acid ester, titanium oxide, talc, dibasic calcium phosphate or its hydrate, corn starch, magnesium aluminometasilicate and sodium lauryl sulfate.

5. A granule formulation for oral administration according to Claim 4, wherein said granule formulation is obtained by mixing together cariprazine hydrochloride, a diluent and a binder, granulating the mixture, then mixing the granulated product in a fluidizer.
